# EUROPEAN PATENT APPLICATION

(11) **EP 2 156 802 A2**
(43) Date of publication of application: **24.02.2010**
(21) Application number: 09165342.8
(22) Date of filing: 13.07.2009
(51) Int. Cl.: A61B 18/14, A61B 18/00, A61B 17/29

(54) **MIS electrosurgical handpiece**

(30) Priority: 18.08.2008 US 228838
(71) Applicant: Ellman, Alan G., Oceanside, New York 11572 (US)
(72) Inventor: Ellman, Alan G., Oceanside, New York 11572 (US)
(74) Representative: Cloughley, Peter Andrew

(57) **Abstract**

An electrosurgical handpiece comprising a squeezable handle connected to extend and retract monopolar or bipolar electrodes from a rigid tubular member. With bipolar electrodes, they comprise active separable distal ends and connecting links configured such that when the handle is unsqueezed, the active distal ends protrude from the distal end of the first tubular member and are adjacent one another, and when the handle is fully squeezed, the active distal ends are fully extended outwardly from the first tubular member and separate, and when the handle is relaxed but still partially squeezed, the active distal ends come together and touch first at their extreme ends and then touch over a broader area in a position to grasp tissue for receiving electrosurgical currents. Preferably, the handle members are rigid and pivotable at midpoints to form handle portions extending both below and above the common axis of the body sections with the above handle portions joined at a fulcrum to provide a mechanical advantage reducing the pressure needed to activate the handpiece.

## Description

This invention relates to a surgical handpiece, and in particular to a handpiece employing electrosurgery especially for performing spinal and related surgical procedures.

### BACKGROUND OF THE INVENTION

A bipolar electrosurgical handpiece is described in US patent No. 6,231,571, the contents of which are herein incorporated by reference, an example of which is known commercially as the Trigger-Flex Bipolar System and is available from Elliquence LLC of Oceanside, NY. The handpiece comprises an elongated rigid tube within which is housed extendable electrosurgical electrodes, preferably of the bipolar type. By special construction of the distal end of the electrodes, such as by the use of memory metal, when the handle is squeezed the bipolar electrodes, whose spacing is fixed, are extended from their tube and bent in accordance with the presetting of the memory metal. Typically, such an electrosurgical handpiece is employed with a cannula for minimally invasive surgical (MIS) procedures.

There are certain procedures in which it is desired for the bipolar electrodes to follow a certain path allowing the electrodes to grasp certain tissue before electrosurgical currents are supplied. Examples are general pin-point coagulation in all delicate neurosurgical procedures, transphenoidal surgery, and in certain cases of devascularization of tumors and debulking of lesions/tumors.

Reference is also made to prior Patent No. 7,101,370, the contents of which are herein incorporated by reference, which also describes a similar handpiece but with a monopolar electrode. This handpiece uses a squeezable handle connected to and across spring biased slidable bodies such that, when the handle is unsqueezed, the bodies assume a first position relative to one another, and when the handle is squeezed, the bodies assume a second position relative to one another extending an active electrosurgical electrode slidingly mounted within a tubular member from its distal end. It is desirable to improve the operating mechanism of such a device also useful in MIS procedures.

### SUMMARY OF THE INVENTION

An object of the invention is an improved electrosurgical handpiece for use in performing MIS procedures.

Another object of the invention is an improved electrosurgical handpiece adapted for grasping tissue in a particular fashion.

A further object of the invention is an electrosurgical handpiece of the type described which is easier to use and improves physician visibility of the surgical site.

In accordance with one aspect of the invention, a novel electrosurgical handpiece comprises an elongated rigid tubular member housing extendable bipolar electrodes, with the tubular member configured to fit within and be extended down a standard sized cannula in a MIS procedure. Squeezable handles support the tubular member and are configured such that when the handles are squeezed, the active electrode ends are extended out through the cannula end and opened, i.e., spread apart. When the handles are released, the electrode ends are pulled back into the cannula and forced to close.

A feature of the invention is the configuration of the tips of the bipolar electrode ends, which are formed into flat opposing surfaces, and the relationship of their connecting links to the tubular member. The connecting links are configured such that, as the handles are released, when they first are forced to close as they withdraw into the tubular member, the distal ends of the electrode tips touch first. As they continue to withdraw into the tubular member and to close, the flat surfaces are pressed up against one another. This action ensures that any tissue grasped as the tips close is held securely in the tips and not squeezed out.

As another feature of the invention, the electrode tips can be arranged to extend out in a straight line along an extension of the elongated tube axis, or bent off the axis in order to reach tissue portions not readily accessible from a straight line extension.

The housed bipolar electrodes as in the referenced patents are electrically active and are capable when energized of applying electrosurgical currents to grasped human tissue with the result that a void or cavity or tunnel can be formed in the tissue or bleeders sealed. Any tissue removed may then be easily aspirated via a suction port connected to the handpiece.

Preferably, radio-frequency (RF) electrosurgical currents, in a frequency range preferably above 3 MHz, with 4 MHz being preferred, are employed. It is believed that 4 MHz radiofrequency energy has been proven to be a self-limiting, minimal penetration energy source capable of precise tissue interaction. Thus, electrosurgical instruments that emit 4 MHz radiofrequency currents will be attractive to spinal or other surgeons needing to produce controlled tissue modulation efficiently and safely. Since lateral heat is typically not a byproduct of 4 MHz RF currents, damage to surrounding tissue can be minimized or avoided.

In accordance with another aspect of the invention, the handle activator of the handpiece comprises two axially spaced rigid members pivoted on and extending below the handpiece body, the two members also extending above the handpiece body and joining as levers at a fulcrum.

Preferably, the rigid members are pivoted at midpoints each on one of two relatively movable bodies of the handpiece, their relative position determining the position of the active part of the electrosurgical electrode. In the embodiment described in the referenced '370 patent, the relative position of the movable bodies extends and retracts the active end of the electrode. Also, in this embodiment, the movable bodies are axially spaced and aligned, with one connected to a fixed part of the handpiece and the other connected to the extendable electrode of the handpiece. The invention however is not limited to such arrangements. The bodies while preferably axially aligned need not be axially-spaced, but can also be coaxial. Also, the action obtained when the bodies are moved need not be limited to extension and retraction of an electrode, but can also involve changing the position of an already extended electrode or modifying the position of parts of an extended electrode, such as opening the jaws of an electrode.

Preferably, the handle sections extending above the handpiece body form an angle below 180°. Preferably, the angle is sufficiently large as to maintain the sections close to the main body and thus less likely to impede the surgeon's view of the surgical site.

The various features of novelty which characterize the invention are pointed out with particularity in the claims annexed to and forming a part of this disclosure. For a better understanding of the invention, its operating advantages and specific objects attained by its use, reference should be had to the accompanying drawings and descriptive matter in which there are illustrated and described preferred embodiments of the invention, like reference numerals or letters signifying the same or similar components.

### BRIEF DESCRIPTION OF THE DRAWINGS

Fig. 1 is an enlarged plan view from the side of one form of electrosurgical handpiece in accordance with the invention in its relaxed position shown schematically connected to an electrosurgical generator;
Fig. 2 is a top view of the electrosurgical handpiece of Fig. 1 also in its relaxed position;
Fig. 3 is an enlarged top view of the protruding electrode ends of the electrosurgical handpiece of Fig. 2 in the relaxed non-squeezed handle position shown in Fig. 1;
Fig. 4 is a side view of the electrosurgical handpiece of Fig. 1 shown in its fully squeezed handle position;
Fig. 5 is an enlarged top view of the protruding electrodes of the electrosurgical handpiece of Fig. 4 in its fully-squeezed position;
Fig. 6 is a side view of the electrosurgical handpiece of Fig. 1 shown in its middle or partially relaxed handle position;
Fig. 7 is an enlarged top view of the protruding electrodes of the electrosurgical handpiece of Fig. 6 in the partially-relaxed handle position;
Fig. 8 is a side view of the electrode and its connecting link for the electrosurgical handpiece of Fig. 1 with a straight electrode;
Fig. 9 is a top view of the electrode and its link of Fig. 8;
Fig. 10 is a cross section along the line 10-10 of Fig. 9.
Fig.11 is a side view of a variant of the electrosurgical handpiece of Fig. 1 in its squeezed position in which the electrode is angled;
Fig. 12 is a side view of the electrode and its connecting link for the electrosurgical handpiece of Fig. 11 with an angled electrode;
Fig. 13 is a top view of the electrode and its link of Fig. 12;

### DETAILED DESCRIPTION OF THE PREFERRED EMBODIMENTS

The present invention is an improvement of the electrosurgical apparatus described in US patents Nos. 6,231,571 and 7,101,370. In the referenced patents, an electrosurgical handpiece is described in which an elongated rigid tube is fixed to the front end handpiece body to which is affixed the front handle portion. Extendable within the rigid tube is an elongated electrode connected to the rear end handpiece body to which is affixed the rear handle portion. When the handles are squeezed, the electrode is extended and bends in a direction preset into the electrode metal. An incorporated compression spring keeps the two body parts apart. The present invention employs the same basic construction except for the handle configuration and the configuration of the electrodes so that when extended and retracted they follow a different path.

Referring now to the drawings, an electrosurgical handpiece 10 in accordance with the invention comprises an elongated rigid tube 12 affixed to the front end body section 14. Inside the rigid tube 12 extends connected to active bipolar electrode ends 16 is an elongated link 18 (shown in dashed lines) which in turn is fixed to the rear end body section 20, which telescopes within the front end section 14. Across the two body sections 14, 20, biased apart by an internal spring shown schematically at 21, is pivotably 22 mounted the front 24 and rear 26 handle portions, which also pivotably connect 28 at the top. The handle configuration disclosed shows that the handle portions above their pivots to the body are angled closer to the body to improve surgeon visibility of the surgical site while maintaining low force activation.

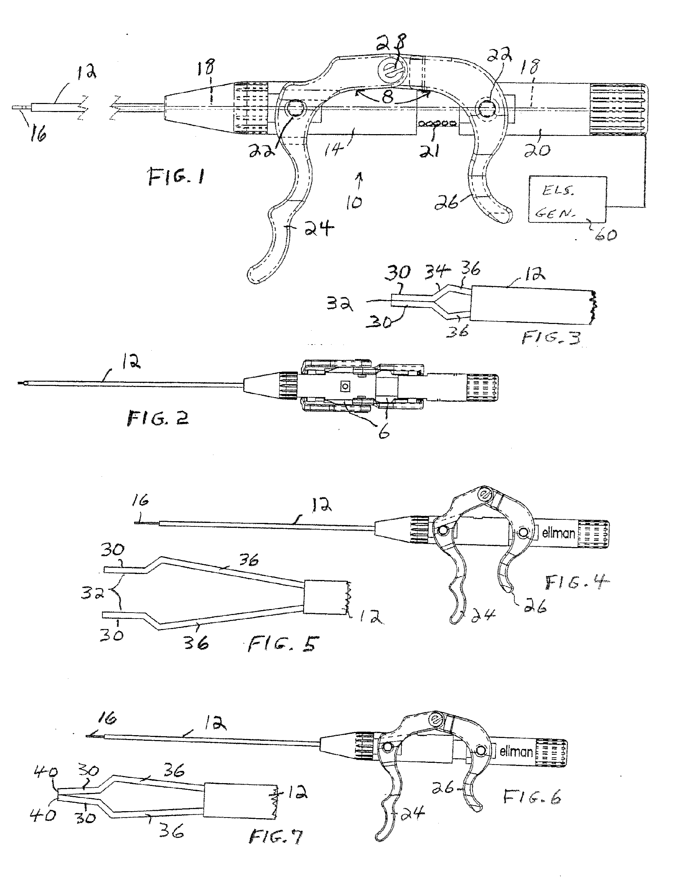

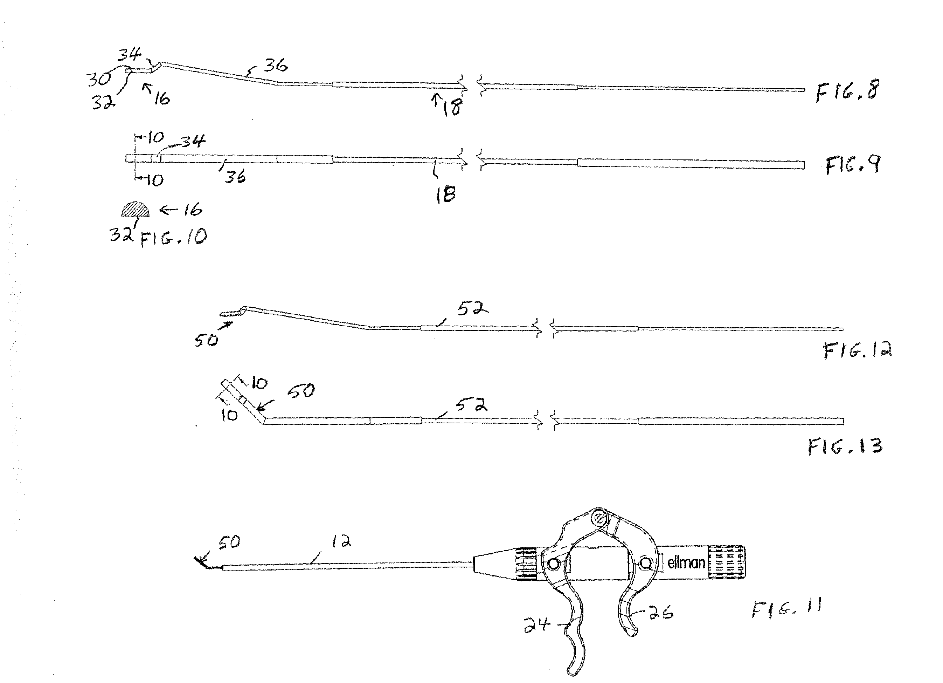

The handpiece is typically bipolar with two extended electrodes between which the electrosurgical currents are concentrated. One electrode 16 and its extended link 18 is shown in Figs. 8-10. Its companion electrode (not shown) is a mirror image of the one shown. The right end of the shank is connected to the rear body section 20, so that when the handles are squeezed the electrode end 16 at the left is extended out of the rigid tube 12. A feature of the invention is the configuration of the electrode 16 and its link 18. As shown in Fig. 8, the left end or distal portion 16 has a short straight flat section 30 with facing surfaces 32 of the active electrode ends that are parallel, electrically bare, and in full contact when the handpiece is in its relaxed position, shown in Fig. 3. Back of the distal section 30 the electrode has a short angled section 34 (at about a 45° angle) followed by a longer straight section 36 that extends toward the center axis of the link. As an example, not to be considered limiting, the rigid tube 12 has an inside diameter of about 2.08 mm (0.083 inches), the overall length of the electrode with its link is about 220 mm, the short parallel section 30 in front is about 3 mm long, the following angled short section 34 is about 1.3 mm long, the longer section 36 returning to the axis is about 15 mm long forming an angle of about 9° where it intersects the axis. The thickness of the straight thicker central section of the link is about 0.5 mm. so when the two bipolar electrodes fill the rigid tube, they occupy about 1/2-2/3 of the internal space. The peak where the short angle section 34 meets the longer straight section 36 is about 3 mm above the electrode axis. As a result of this configuration, when one of the electrode pair 16 is inside the rigid tube 12 in the relaxed position shown in Fig. 3, the longer straight section 36 of each electrode half bears against the inside wall of the rigid tube 20 forcing the flat distal ends 30 together with their facing surfaces 32 in full contact. At that position, about 2/3 of the tapered longer sections 36 are inside the rigid tube 12.

When the handles are fully squeezed as shown in Figs. 4 and 5, the electrode ends 30 are extended out about 4/5 of the length of the longer sections 36. Due to the closer spacing of the preset tapered sections 36 still remaining inside the rigid tube, the distal ends 30 spread apart but the opposed surfaces 32 due to the geometry still remain essentially parallel. Now, as shown in Fig. 7, as the hand pressure on the handles 24, 26 relaxes, the electrodes 18 due to the internal spring 21 pressure are forced back into the rigid tube 12, and the internal wall pressure on the sections 36 cause the distal ends 30 to approach one another. The configuration of the electrodes are such that the pressure of the rigid tube 12 on the retracting tapered sections 36 forces the extreme electrode ends 40 toward one another faster than the rearward sections with the result that the extreme ends 40 touch first (Fig. 7), before the rest of the front flat sections. In this position, about ½ of the tapered straight sections 36 remain within the rigid tube 12. Then, as the handles are further relaxed, the remaining parts of the front distal section gradually come together until the position shown in Fig. 3 is restored with the front sections again in full contact over their full facing surfaces 32.

To summarize, when the handles are released, the electrode links 18 are pulled back into the rigid tube acting as a cannula and forced to close. But the tips 30, 34 are bent so that when they first begin to close, the extreme ends 40 touch first, and as they continue to close, the flat faces 32 are finally pressed up against one another. This action is extremely important because it allows the surgeon to position the open ends with their extreme tips exactly at the tissue to be grasped and helps to ensure that the tissue is held securely in the tips and not squeezed out during the further closing action.

In the preferred embodiment, the distal end sections 16 have a semi-circular configuration as illustrated in Fig. 10 with the flat active surface shown at 32.

In the embodiment described above, the active electrode ends 16 extend straight out parallel to the rigid tube axis. In the embodiment illustrated in Figs. 11-13, the active electrode ends are shown at 50 connected as before to an extended link 52. The bipolar active electrode ends not only spread apart (not shown) as they are extended but also angle off to the left as shown at 50. They could just as easily angle off to the right if desired. This bending action is similar to that obtained with the handpiece of the referenced patents and is obtained by simply pre-bending the electrodes so that upon their release from the confining action of the rigid tube, they will automatically spread apart as well as angle off to the side as indicated.

As in the referenced patents/application, when the tissue has been grasped, then the surgeon can apply to the tissue via the electrode ends electrosurgical currents by the usual footswitch connected to a conventional electrosurgical generator 60 (Fig. 1), also available from Elliquence LLC of Oceanside, NY.

While the instrument of the invention is especially useful for spinal procedures, it is not limited to such uses and it will be understood that it can be employed in any electrosurgical procedure employing a cannula in MIS.

The reader is directed to the referenced prior patents for a more detailed description of a flexible tip handpiece which will assist in understanding the improvements offered by the present application. Since the present application otherwise makes use of the same teachings of the prior patent, it was felt unnecessary to repeat in the body of this specification many of the details present in the contents of the prior patents. The present description will be confined mainly to the modifications in the handpiece construction that allow for increased control by the user over the extendable tip. In addition, the construction of the present invention can provide both bipolar and unipolar operation separately or in the same handpiece, and can use the same constructions described in the prior patent for providing the extendable and retractable straight and/or curved active electrode tips, as well as many of the details for providing a flexible end or a straight end with a curved extendable electrode, including use in the various medical procedures described in the prior patent and known to others in this art in which electrosurgical currents are used to modulate patient tissue, meaning to cut, ablate, shrink, and/or coagulate tissue.

The novel handle assembly comprises the front handle part 24 and the rear handle part 26 axially spaced from the former, both of which extend below the axis 18 of the handpiece when the latter is held in its upright position as illustrated in Fig. 1. The upper end of each handle part is bifurcated 6 just below its respective pivot point 22, and then the bifurcated handle parts extend upward above the axis 18 and are angled toward one another and joined at a fulcrum point 28. Actually, the latter is an axis due to the bifurcated upper section. The joined handle parts define an inner obtuse angle 8. The pivotable mounting of the handle parts on their respective pivot pins are freely rotatable, and their junction at the fulcrum point 28 is also freely rotatable. Each of the handle parts 24, 26 are rigid members.

The handle assembly of the invention 24, 26 is configured so that the user can use different fingers of one hand to squeeze the two handle parts, with fingers on both sides of the handpiece handle, and with the handpiece upright or inverted or upside down. This is possible because of the concave-shaped regions provided on the handle parts. Specifically, four concave-shaped sections are provided, one on the rear handle part facing rearward, two on the bottom side of the front handle part facing frontward, and one on the top side of the front handle part also facing frontward. Each of the front and rear handle parts, designated generally 24 and 26, are rigid members from their lowest points, as viewed in Fig. 1, to where they join at the fulcrum axis 28. In comparison with the referenced patent, which is a whole hand articulating device, the construction of the invention is meant for 4, 3, or 2-finger control. Using just fingertips to articulate the handle allows for finer feedback, as there is a higher nerve density at fingertips as opposed to the middle or base of fingers. Used upside down or sideways where space limitations are imposed provides no reduction in performance or ergonomics.

The combination of the pivot points 22 and the fulcrum point 28, for the dimensions shown in the drawings, provides the user with an approximate mechanical advantage of about 2:1, which depends on the distance of the fulcrum 28 to each of the pivot points 22 as well as to the bottom ends of the handle members. The mechanical advantage can be reduced or increased by adjusting the lengths of the arm portions that make up the handle. For example, the angle 8 can be varied from about 60-180 °. Preferably, it is between 140° and 180°. The larger the angle, the shallower is the structure. The respective lengths of the various arm members can be varied from about 1 inch to about 2 inches. The shorter the arm lengths, the more convenient the instrument is to use, but the mechanical advantage is reduced. Similarly, the longer the arm lengths, the less convenient the instrument is to use, but the mechanical advantage is increased. It will be appreciated that, in the absence of the fulcrum attachment (it should be understood that point 28 is designated as the fulcrum to distinguish it from the two pivot points 22), since each handle part 24, 26 as a rigid member is pivoted at an approximate midpoint on a pivot pin 22 of each of the front and rear body sections 14, 20, respectively, each will freely rotate about its respective pivot point. The pivot points 22 incidentally are in the same plane as the handpiece axis 18 or centerline. Similarly, the two handle parts 24, 26 each rotate freely where they join about the fulcrum 28. It is this unique arrangement that provides the increased control of the electrode tip when extended, which not only makes the device easier to squeeze, but also de-magnifies the user input for finer position control of the electrode tip. In general, the movement of the handle parts is proportionally larger than the corresponding movement of the electrode tip. Modifications are also possible with varying degrees of de-magnification by altering the locations of the concave finger rests and fulcrum point relative to the pivot points. Another way of understanding the mechanism is to note the value of the inside obtuse angle, designated 8, formed by the two handle parts where they meet at the fulcrum 28. As the handle is squeezed, that inside angle 8 decreases (Figs. 4 and 6), with the result that the corresponding amount of electrode tip extension also gradually reduces as the axial distance between the handle parts 64, 66 reduces.

The new handle configuration with the large angle shown importantly also reduces the overall height of the instrument which allows more freedom when in tight positions, especially noticeable when the instrument is close to the user's body. Moreover, the lower profile of the handpiece viewed along the top means less likelihood of obstruction of the surgeon's view of the surgical site. This is achieved by the large angle 8, preferably close to 180°, between the handle upper sections joined at the fulcrum, which maintains those handle sections close to the handpiece body, without jeopardizing the important mechanical advantage obtained.

As used herein, by "axial" is meant parallel to the long axis of the handpiece and electrode (horizontal in Fig. 1). By "lateral" is meant transverse to the long axis.

Once the surgeon has positioned the working end of the handpiece with respect to the tissue to be operated on, he or she can, if desired, then activate the electrosurgical apparatus causing a discharge of bipolar currents between the bare electrode ends 30 capable of causing ablation, shrinkage, or excision of tissue, or cauterization of a blood vessel in the usual way. Other usable mechanical or electrical structures following the teachings of the prior patents will be appreciated by those skilled in this art. Both aspects of the invention are combined in the handpiece illustrated in Fig. 1 and cooperate to provide the improved electrosurgical performance.

In all embodiments, the tubular housing 18 can be plastic, such as ABS or DELRIN, or of insulated relatively stiff metal that will not bend.

While the invention has been described in connection with preferred embodiments, it will be understood that modifications thereof within the principles outlined above will be evident to those skilled in the art and thus the invention is not limited to the preferred embodiments but is intended to encompass such modifications.

## Claims

1. An electrosurgical handpiece comprising:
(a) a first main body,
(b) a second main body axially aligned with and coupled to the first main body,
(c) a squeezable handle connected to the first and second main bodies such that, when the handle is unsqueezed, the first and second main bodies assume a first relaxed position relative to one another, and when the handle is fully squeezed, the first and second main bodies assume a second fully squeezed position relative to one another, and when the handle is partially squeezed, the first and second main bodies assume a third partially squeezed position relative to one another,
(d) a first tubular member having a distal end and a second end connected to one of the first and second main bodies,
(e) a bipolar electrosurgical electrode comprising active separable distal ends and connecting links connected to the other of the first and second main bodies and extending within the tubular member,
**characterized in that**:
(f) the active distal ends and their connecting links are configured such that:
i) when the handle is unsqueezed the active distal ends protrude from the distal end of the first tubular member and are adjacent one another, and
ii) when the handle is fully squeezed the active distal ends are fully extended outwardly from the first tubular member and separate, and
iii) when the handle is relaxed but still partially squeezed, the active distal ends come together in a position to grasp tissue for receiving electrosurgical currents.

2. An electrosurgical handpiece as set forth in claim 1, wherein when the handle is relaxed but still partially squeezed, the active distal ends come together and touch first at their extreme ends and then touch over a broader area in a position to grasp tissue for receiving electrosurgical currents.

3. An electrosurgical handpiece as set forth in claim 2, wherein the active distal ends extend straight outwardly in line with the first rigid tubular member when the handle is fully squeezed.

4. An electrosurgical handpiece as set forth in claim 2, wherein the active distal ends extend outwardly and are bent at an angle to the first rigid tubular member when the handle is fully squeezed.

5. An electrosurgical handpiece as set forth in claim 2, wherein the active distal ends comprise straight portions with facing flat surfaces.

6. An electrosurgical handpiece as set forth in claim 5, wherein the link sections following the active distal ends comprise short straight portions angled outwardly relative to the distal ends, and wherein the link sections following the short straight portions comprise longer straight portions angled inwardly relative to the distal ends.

7. An electrosurgical handpiece as set forth in claim 5, wherein the link sections bear against the inside surface of the rigid tubular member.

8. An electrosurgical handpiece as set forth in claim 5, wherein the link sections following the active distal ends comprise long straight portions angled inwardly relative to the distal ends and bearing against the inner surface of the rigid tubular member.

9. The electrosurgical handpiece of claim 1 in combination with an RF generator generating high frequency currents at a frequency of about 4 MHz.

10. An electrosurgical handpiece comprising:
(a) a first main body,
(b) a second main body axially aligned with and coupled to the first main body,
(c) a squeezable handle connected to the first and second main bodies such that, when the handle is unsqueezed, the first and second main bodies assume a first axial position relative to one another, and when the handle is squeezed, the first and second main bodies assume a second axial position relative to one another,
(d) means for biasing the first and second main bodies into their first position,
(e) a first member having a first end and a second end and connected to one of the first and second main bodies,
(f) an active electrosurgical electrode connected to the first member and movable into an active position at the second end of the first member and connected to the other of the first and second main bodies, the electrosurgical electrode having a contact end and at least one electrically active end adjacent the second end of the first member,
(g) electrical terminal means at one of the first and second main bodies and electrically connected to the electrically active end of the electrosurgical electrode, whereby, when the electrical terminal means is activated and the handle squeezed, the first and second main bodies assume their second position and the electrically active end of the electrode can be moved into its active position at the second end of the first member and is capable of supplying electrosurgical currents when applied to a patient,
**characterized in that**:
the squeezable handle comprises:
(h) a first rigid arm member pivotably connected at a midpoint to the first main body and having a first arm portion extending below its connected midpoint and a second arm portion extending above its connected midpoint,
(i) a second rigid arm member pivotably connected at a midpoint to the second main body and having a first arm portion extending below its connected midpoint and a second arm portion extending above its connected midpoint,
(j) the second arm portions of the first and second arm members being joined together above their connected midpoints to form a pivotable fulcrum axis and form at the fulcrum an angle such that obstruction of a user's view of the active electrosurgical electrode is reduced.

11. An electrosurgical handpiece as claimed in claim 10, wherein the first and second arm portions are provided with concave portions configured to receive fingers of a user.

12. An electrosurgical handpiece as claimed in claim 10, wherein the second arm portions of the first and second arm members form at the fulcrum an angle of about 60-180°.

13. An electrosurgical handpiece as claimed in claim 10, wherein the second arm portions of the first and second arm members form at the fulcrum an angle of about 140-180°.

14. An electrosurgical handpiece as claimed in claim 10, wherein the second arm portions of the first and second arm members form at the fulcrum an angle of just below 180°.

15. An electrosurgical handpiece as claimed in claim 10, wherein the first and second arm portions are dimensioned to provide a mechanical advantage of about 2:1 for the user.
